(19) Europäisches Patentamt
European Patent Office
Office européen des brevets

(11) Publication number: **0 002 624**
**B1**

# (12) EUROPEAN PATENT SPECIFICATION

(45) Date of publication of patent specification: **14.10.81**

(21) Application number: **78300863.4**

(22) Date of filing: **19.12.78**

(51) Int. Cl.[3]: C 07 D 223/16, A 61 K 31/55 //C07D209/38, C07D265/26, C07C131/00, C07C101/54, C07C69/76, C07C39/34, C07C21/24, C07C79/46, C07C79/22

(54) **2-Benzazepine compounds, processes for their preparation and pharmaceutical compositions containing them.**

(30) Priority: **21.12.77 US 862892**

(43) Date of publication of application:
**27.06.79 Bulletin 79/13**

(45) Publication of the grant of the European patent:
**14.10.81 Bulletin 81/41**

(84) Designated Contracting States:
**BE CH DE FR GB IT LU NL SE**

(56) References cited:
US - A - 3 828 096

(73) Proprietor: **SMITHKLINE CORPORATION**
**1500 Spring Garden Street P.O. Box 7929**
**Philadelphia, Pennsylvania 19101 (US)**

(72) Inventor: **Bondinell, William Edward**
**46 Curtis Avenue**
**Cherry Hill New Jersey 08002 (US)**
Inventor: **Pendleton, Robert Grubb**
**720 Cornelia Place**
**Philadelphia Pennsylvania 19118 (US)**

(74) Representative: **Hargreaves, Gerald Henry, Dr.**
**P.O.Box 39**
**Welwyn Garden City Hertfordshire AL7 1EY (GB)**

**EP 0 002 624 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

2-benzazepine compounds, processes for their preparation and pharmaceutical compositions containing them

This invention relates to 2-benzazepine compounds which inhibit the enzyme phenylethanolamine N-methyl-transferase, processes for the preparation of such compounds, and pharmaceutical compositions containing them.

Epinephrine is a hormone, synthesized in the adrenal medulla, which is released into the blood stream in response to stress and produces physiological changes which serve to prepare the animal to cope with stress. For example, epinephrine produces anxiety, an increase in blood pressure, acceleration of heart rate, and increase in cardiac output. These changes are detrimental in individuals with certain disease conditions such as angina pectoris, myocardial infarction and anxiety neuroses.

Phenylethanolamine N-methyltransferase catalyzes the final step in the biosynthesis of epinephrine, that is the transfer of a methyl group from S-adenosylmethionine to norepinephrine to produce epinephrine.

The compounds of this invention inhibit phenylethanolamine N-methyl-transferase and thus reduce the formation of epinephrine. They are therefore of value in situations where there is overproduction of epinephrine or where epinephrine production is detrimental.

The compounds of this invention are of formula:

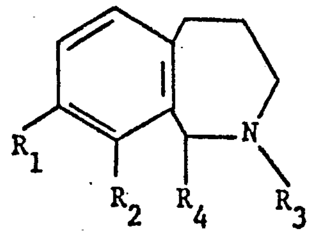

Formula I

where $R_1$ and $R_2$, which are the same or different, are hydrogen, chloro, bromo, fluoro, iodo, trifluoromethyl, amino, nitro, hydroxy, sulfamyl, methylthio, methylsulfinyl, methylsulfonyl, trichloromethylsulfinyl, trichloromethylsulfonyl, trifluoromethylthio, trifluoromethylsulfinyl, trifluoromethylsulfonyl, lower alkyl and lower alkoxy of from 1 to 3 carbon atoms each, provided that $R_1$ and $R_2$ are not both hydrogen; and $R_3$ and $R_4$, which are the same or different, are hydrogen or lower alkyl of from 1 to 3 carbon atoms; and pharmaceutically acceptable acid addition salts thereof.

Preferred compounds of this invention are represented by formula I above when $R_1$ and $R_2$ are chloro, trifluoromethyl or sulfamyl, and $R_3$ and $R_4$ are both hydrogen.

An advantageous compound of this invention is 8,9-dichloro-2,3,4,5-tetrahydro-1H-2-benzazepine.

The compounds of this invention can be prepared by the following procedure:

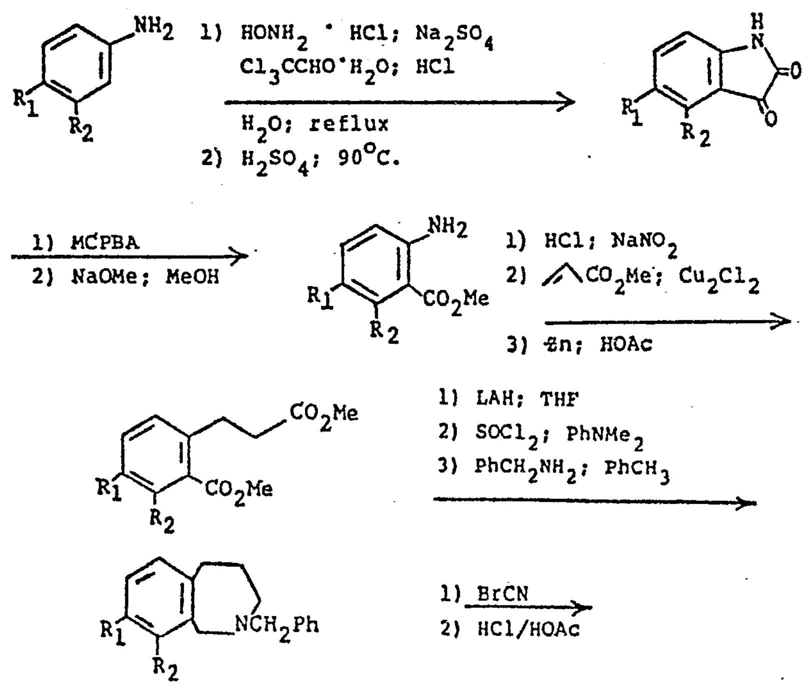


II

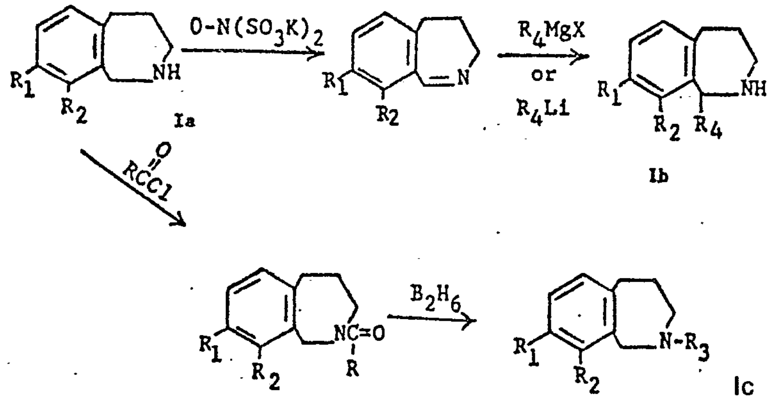


where

$R_1$, $R_2$, $R_3$ and $R_4$ are as defined above.

According to the above procedure, a substituted aniline is reacted with chloral hydrate, hydroxylamine hydrochloride, sodium sulfate, and hydrochloric acid, and then with concentrated sulfuric acid, to yield a 4-, 5-, or 4, 5-substituted isatin. The isatin is treated with m-chloroperbenzoic acid, and then with sodium methoxide, to yield the 5-, 6- or 5,6-substituted methyl anthranilate which in turn is converted into the correspondingly substituted methyl 3-[(2-carboxymethoxy)phenyl]propionate by treating the anthranilate with sodium nitrite, methyl acrylate and cuprous chloride followed by reduction.

The propionate is reduced to the corresponding diol with lithium aluminium hydride or diborane, and cyclization is carried out by treating the diol with N,N-dimethylaniline, thionyl chloride, and then with benzylamine to yield the 8-, 9- or 8,9-substituted 2-benzyl-2,3,4,5-tetrahydro-1H-2-benzazepine. This 2-benzazepine is then treated with cyanogen bromide and acid to give the desired 8-, 9- or 8,9-substituted 2,3,4,5-tetrahydro-1H-2-benzazepine. Alternatively, the 2-benzyl group can be removed by catalytic hydrogenation.

The N-substituted compounds of formula I are formed by converting the N-unsubstituted 2-benzazepine into an amide by reaction with a lower alkanoyl halide, and then reducing the amide, for example with diborane, to the desired N-substituted 2-benzazepine.

When a compound of Formula I is desired where $R_4$ is other than hydrogen, the benzazepine is first oxidized to the imine as shown above, and then treated with an organometallic agent such as a lower alkyl magnesium halide or a lower alkyl lithium to give the desired product.

Pharmaceutically acceptable acid addition salts of the compounds of Formula 1 can be formed with organic and inorganic acids by methods known to the art. The base of Formula I can be reacted with an organic or inorganic acid in a water miscible solvent, such as acetone or ethanol, with isolation of the salt by concentration and cooling, or in a water immiscible solvent, such as ethyl ether or chloroform, with the desired salt separating directly. Examples of salts of this invention are the maleate, fumarate, benzoate, ascorbate, pamoate, succinate, bismethylenesalicylate, methanesulfonate, ethanedisulfonate, benzenesulfonate, acetate, propionate, tartrate, salicylate, citrate, gluconate, lactate, malate, mandelate, cinnamate, citraconate, aspartate, stearate, palmitate, itaconate, glycolate, p-aminobenzoate, glutamate, theophylline acetates, hydrochloride, hydrobromide, sulfate, cyclohexylsulfamate, phosphate and nitrate salts.

7-Chloro-2,3,4,5-tetrahydro-1H-2-benzazepine is known in the art, *J. Chem. Soc. Perkin I, 782—3 (1973)*

The activity of the compounds of this invention is demonstrated by inhibition of phenylethanolamine N-methyltransferase *in vitro* by the assay procedure described by Pendleton and Snow, *Molecular Pharmacology, 9:718—725 (1973)* at various compound concentrations.

The following are results comparing 2-benzazepine compounds of the invention with the 7-chloro prior art compound.

Table 1 — In Vitro % Inhibition

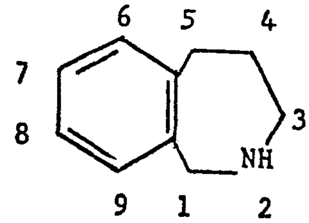

| Compound | $10^{-4}$ M | $10^{-6}$ M |
|---|---|---|
| 7-chloro | 96 | 21 |
| 8-chloro | 99 | 76 |
| 8,9-dichloro | 99 | 96 |

The results of this test clearly demonstrate that both the 8-chloro and the preferred compound of this invention, 8,9-dichloro-2-benzazepine, are greater inhibitors of PNMT than the known 7-chloro compound.

In addition, the activity of the compounds of this invention is demonstrated *in vivo* by their administration to mice at 50 mg./kg. per day for seven consecutive days. Male mice were dosed orally with either drug or vehicle control on a twice-a-day basis for seven consecutive days. On the morning of the next day they were again dosed, and two hours later they were sacrificed by decapitation. The adrenal glands were then removed and analyzed fluoremetrically for both epinephrine and norepinephrine content. A compound is considered active as a PNMT inhibitor if it significantly (at least $p<0.05$) decreases the adrenal epinephrine/norepinephrine ratio. (R. G. Pendleton *et al., J. Pharmacol Exp. Ther. 190:551—562, 1974* and R. G. Pendleton *et al., J. Pharmacol. Exp. Ther. 197:623—632, 1976).* The preferred compound of this invention 8,9-dichloro-2,3,4,5-tetrahydro-1H-2-benzazepine significantly lowered the adrenal epinephrine/norepinephrine ratio ($p<0.001$) at a daily dose of 25 mg./kg.

The invention also provides pharmaceutical compositions which comprise a pharmaceutical carrier and, as active ingredient, a 2-benzazepine compound of Formula 1 or a pharmaceutically acceptable salt thereof. The active ingredient will be present in the compositions in an effective amount to inhibit phenylethanolamine N-methyltransferase.

Preferably, the compositions of this invention contain an active ingredient of Formula 1 in an amount of from about 50 mg. to about 1000 mg., advantageously from about 100 mg to about 500 mg., per dosage unit.

The pharmaceutical carrier can, for example, be a solid or a liquid. Examples of solid carriers are lactose, magnesium stearate, terra alba, sucrose, talc, stearic acid, gelatin, agar, pectin or acacia. The amount of solid carrier can be varied widely, but preferably it will be from about 25 mg to about 1 gm. Examples of liquid carriers are syrup, peanut oil, olive oil, sesame oil, propylene glycol, polyethylene glycol (mol. wt. 200—400), and water. The carrier at diluent can include a time delay material well known to the art, for example glyceryl monostearate or glyceryl distearate, alone or with a wax.

A wide variety of pharmaceutical forms can be employed, for example the compositions can take the form of tablets, capsules, powders, troches, lozenges, syrups, emulsions, sterile injectable liquids, or liquid suspensions or solutions. The pharmaceutical compositions can be prepared by conventional techniques involving procedures such as mixing, granulating and compressing, or dissolving the ingredients as appropriate to the desired form of composition.

Phenylethanolamine N-methyltransferase can be inhibited by administering to an animal a 2-benzazepine compound of the invention in an amount sufficient to inhibit phenylethanolamine N-methyltransferase.

Preferably, the compounds of the invention are administered in conventional dosage unit forms prepared by combining an appropriate dose of the compound with standard pharmaceutical carriers.

Preferably, compounds of the invention will be administered in a daily dosage regimen of from about 100 mg. to about 2,000 mg. most preferably from about 200 mg. to about 1000 mg. .

Advantageously, equal doses will be administered, preferably two to three times per day. When the administration is carried out as described above, inhibition of phenylethanolamine N-methyltransferase is produced.

The route of administration of the pharmaceutical compositions of this invention is internal, either parenterally or preferably orally, in an amount to produce the desired biological activity.

The following Examples illustrate the invention.

Example 1

80 g. of 3,4-dichloroanline (0.5 mole) in concentrated hydrochloric acid (43 ml.) and water (300 ml.) were mixed with 90 g. of chloral hydrate (0.54 mole) in water (1.21), 571 g. of sodium sulfate in water (725 ml.), and 110 g. of hydroxylamine hydrochloride (1.5 mole) in water (500 ml.), heated to reflux, cooled, and filtered to give 3,4-dichloroisonitrosoacetanilide: mp 162—164°C.

360 g. of the isonitrosoacetanilide (1.55 mole) were gradually added to concentrated sulfuric acid (1.51.) stirred at 90°C. The mixture was stirred for 30 minutes, cooled, poured onto crushed ice, and filtered. The filter cake was dissolved in 2N sodium hydroxide, filtered, acidified with acetic acid, and filtered to give 4,5-dichloroisatin: mp 146—248°C.

220 g. of 4,5-dichloroisatin (1 mole) were suspended in ethyl acetate (1.1.) and reacted with 172 g of m-chloroperbenzoic acid (1 mole), stirred, filtered, and washed to yield dichloroisatoic anhydride: mp 268—271°C. d.

150 g. of the anhydride (0.65 mole) were suspended in dry methanol (750 ml.) and reacted with sodium methoxide. The mixture was refluxed 30 minutes until the evolution of carbon dioxide stopped. The mixture was cooled, filtered, acidified with acetic acid, and evaporated to yield methyl 5,6-dichloroanthranilate. The crude anthranilate was converted into the hydrochloride: mp 180—183°C. (sealed tube).

108 g. of methyl 5,6-dichloroanthranilate hydrochloride (0.425 mole) in acetone (200 ml.), concentrated hydrochloric acid (120 ml.) and 120 g of ice were stirred, cooled and treated dropwise with a solution of 31 g. of sodium nitrite (0.45 mole) in water (70 ml.). 40 g. of methyl acrylate (0.47 mole) and 8 g. of cuprous chloride (0.08 mole) were added and the mixture was stirred at 10°C. until nitrogen evolution ceased. The mixture was stirred at 25°C. for one hour, extracted with benzene, and the benzene extracts were washed with saline, dried and evaporated to give methyl 2-chloro-3-[(2-carbomethoxy-3,4-dichloro)phenyl]propionate.

117 g. of the 2-chloro-propionate (0.36 mole) were dissolved in acetic acid (500 ml.), stirred at 90°C., and treated with excess powdered zinc added in small portions until the reaction was complete. The mixture was cooled, filtered, and the filtrate was evaporated and partitioned between chloroform and water. The chloroform extracts were washed with 5% aqueous bicarbonate and evaporated to give methyl 3-[(2-carbomethoxy-3,4-dichloro)phenyl]propionate, b.p. 170—190°C. (3 mm.Hg.).

37 g. of this propionate (0.127 mole) in tetrahydrofuran (150 ml.) were reduced with 8.8 g. of lithium aluminum hydride (0.23 mole) at 0°C for 6 hours and at 25°C for 40 hours, and then cooled to 0°C. Ethyl acetate was added followed by water and then hydrochloric acid, and the mixture was extracted with chloroform to give 3-[(3,4-dichloro-2-hydroxymethyl)-phenyl]propanol.

27 g. of the propanol (0.115 mole) were dissolved in chloroform (360 ml.) and 58.5 g. of N,N-dimethylaniline (0.48 mole), stirred, and cooled to 0°C., 62 g. of thionyl chloride (0.53 mole) were then added over one hour. The mixture was stirred at 3°C for one hour and then at reflux for four hours. The mixture was cooled, poured onto crushed ice, and extracted with benzene. Evaporation gave 3-[(2-chloromethyl-3,4-dichloro)-phenyl]propyl chloride.

30 g. of the propyl chloride (0.11 mole) and 35.5 g. of benzylamine (0.33 mole) in benzene (150 ml.) were refluxed for two days, the solvent evaporated, and heated at 95°C for six hours. The mixture was diluted with water and extracted with toluene to give 2-benzyl-8,9-dichloro-2,3,4,5-tetrahydro-1H-2-benzazepine which was purified as the hydrochloride: mp 198—199°C.

3.06 g. of 2-benzyl-8,9-dichloro-2,3,4,5-tetrahydro-1H-2-benzazepine (0.01 mole) stirred in benzene were treated with cyanogen bromide, refluxed, and evaporated to give 2-cyano-8,9-dichloro-2,3,4,5-tetrahydro-1H-2-benzazepine: mp 132.5—134°C.

2.1 g. of 2-cyano-8,9-dichloro-2,3,4,5-tetrahydro-1H-2-benzazepine (0.009 mole) were refluxed with acetic acid (50 ml.) and concentrated hydrochloric acid (5 ml.) for 24 hours, evaporated, and the residue recrystallized from methanol-ether to give 8,9-dichloro-2,3,4,5-tetrahydro-1H-2-benzazepine: mp 266—268°C.

Example 2

Following the procedure of Example 1, the following substituted isatin compounds:

4-bromoisatin
4-chloroisatin
4-chloro-5-methoxyisatin
4-iodoisatin
4-trifluoromethylisatin
5-bromo-4-chloroisatin
5-trifluoromethylisatin
4-chloro-5-methylisatin
5-chloro-4-methylisatin
4,5-dimethylisatin

are used as starting materials to give the following products, respectively:

9-bromo-2,3,4,5-tetrahydro-1H-2-benzazepine
9-chloro-2,3,4,5-tetrahydro-1H-2-benzazepine

9-chloro-8-methoxy-2,3,4,5-tetrahydro-1H-2-benzazepine
9-iodo-2,3,4,5-tetrahydro-1H-2-benzazepine
9-trifluoromethyl-2,3,4,5-tetrahydro-1H-2-benzazepine
8-bromo-9-chloro-2,3,4,5-tetrahydro-1H-2-benzazepine
8-trifluoromethyl-2,3,4,5-tetrahydro-1H-2-benzazepine
9-chloro-8-methyl-2,3,4,5-tetrahydro-1H-2-benzazepine
8-chloro-9-methyl-2,3,4,5-tetrahydro-1H-2-benzazepine
8,9-dimethyl-2,3,4,5-tetrahydro-1H-2-benzazepine

Example 3

Following the procedure of Example 1, the following substituted aniline compound:

3,4-bis-(trifluoromethyl)aniline is used as starting material to give the following product: 8,9-bis-(trifluoromethyl)-2,3,4,5-tetrahydro-1H-2-benzazepine.

Example 4

206 g. of methyl 3-[(2-carbomethoxy)phenyl]propionate (0.93 mole) were dissolved in concentrated sulfuric acid (2.1), stirred at −10°C., and treated with 84 g. of sodium nitrate (1.0 mole). The mixture was stirred for 3 hours as it warmed to 25°C, and it was then poured onto crushed ice and extracted with ether. The ether was washed, dried, and evaporated to give crude methyl 3-[(2-carbomethoxy-4-nitro)phenyl]propionate.

26.7 g. of the propionate (0.1 mole) dissolved in tetrahydrofuran (100 ml.) were stirred and treated with diborane (0.25 M) in tetrahydrofuran (250 ml.). The mixture was refluxed for 16 hours, cooled, treated with methanol and evaporated. The residue was heated for 15 minutes with 10% hydrochloric acid, cooled, extracted with ether, and the ether was evaporated to give 3-[(2-hydroxymethyl-4-nitro)phenyl]propanol.

100 g. of the propanol (0.475 mole) dissolved in pyridine (1.1.) stirred at 0°C. were treated with 120 g. of methanesulfonyl chloride (1.05 mole). The mixture was stirred below 15°C for 4 hours, poured into water, acidified with hydrochloric acid, and extracted with ether to give the dimesylate ester of 3-[(2-hydroxymethyl-4-nitro)phenyl]propanol.

70 g. of the dimesylate (0.19 mole) dissolved in dimethylformamide (600 ml.) were treated with 120 g. of benzylamine (1.12 mole), stirred for 16 hours, diluted with water, and extracted with ether to give crude 2-benzyl-8-nitro-2,3,4,5-tetrahydro-1H-2-benzazepine which was purified by chromatography.

24.4 g of the 2-benzazepine(0.086 mole) in acetic acid (200 ml.) containing 3 g. of 10% palladium-on-carbon were hydrogenated until the theoretical amount of hydrogen was absorbed. The mixture was filtered and the filtrate was evaporated to give 8-amino-2,3,4,5-tetrahydro-1H-2-benzazepine purified as the fumarate, mp: 190—191°C.

14.4 g. of the benzazepine (0.089 mole) in ethyl acetate (150 ml.) containing 14.4 g. of isopropenyl acetate (0.144 mole) were refluxed for 16 hours and evaporated to give 2-acetyl-8-amino-2,3,4,5-tetrahydro-1H-2-benzazepine.

3.0 g. of the benzazepine (0.0147 mole) dissolved in concentrated hydrochloric acid (30 ml.) were stirred at 0°C and treated with a solution of 1.3 g. of sodium nitrite (0.019 mole) in water (5 ml.). The solution of the diazonium salt was added to a stirred suspension of 4.5 g. of cuprous chloride (0.045 mole) in water (5 ml.) and concentrated hydrochloric acid (45 ml.) maintained at −10°C. The mixture was stirred at 25° for one hour, poured onto crushed ice and extracted with ethyl acetate to give 2-acetyl-8-chloro-2,3,4,5-tetrahydro-1H-2-benzazepine.

2.5 g. of the benzazepine (0.0112 mole) were refluxed with 10% hydrochloric acid for 16 hours, cooled, and the solvent was evaporated to give 8-chloro-2,3,4,5-tetrahydro-1H-2-benzazepine hydrochloride: mp: 305—308°C.

Example 5

2.0 g. of 2-benzyl-8-nitro-2,3,4,5-tetrahydro-1H-2-benzazepine (0.007 mole), as prepared in Example 4, were dissolved in toluene (20 ml.), stirred at 5°C., and treated with 2.0 g. of cyanogen bromide (0.018 mole) and stirred for one hour. The mixture was diluted with hexane, filtered, and evaporated to yield 2-cyano-8-nitro-2,3,4,5-tetrahydro-1H-2-benzazepine.

1.4. g. of the cyano compound (0.0065 mole) were refluxed in acetic acid (10 ml.) and concentrated hydrochloric acid (15 ml.) for 24 hours, cooled, evaporated, and purified as the bitartrate salt to give 8-nitro-2,3,4,5-tetrahydro-1H-2-benzazepine bitartrate, mp: 213.5—214.5°C.

Example 6

Following the procedure of Example 4, 2-acetyl-8-amino-2,3,4,5-tetrahydro-1H-2-benzazepine is diazotized with sodium nitrite and hydrochloric acid or sulfuric acid.

The solution of the diazonium salt was treated with sodium fluoborate or with cuprous bromide to give, after hydrolysis, respectively:

8-fluoro-2,3,4,5-tetrahydro-1H-2-benzazepine, and
8-bromo-2,3,4,5-tetrahydro-1H-2-benzazepine

Example 7

Following the procedure of Example 4, substituting equivalent amounts of sulfuric acid for hydrochloric acid in the diazotization step, and adding the solution of diazonium salt to aqueous potassium iodide, gave, after hydrolysis, 8-iodo-2,3,4,5-tetrahydro-1H-2-benzazepine hydrochloride, mp: 320—324°C.

Example 8

16.2 g. of 8-amino-2,3,4,5-tetrahydro-1H-2-benzazepine, as prepared in Example 4, (0.1 mole) and 20 g. of acetic anhydride (0.2 mole) in acetic acid (200 ml.) are stirred at 25°C for 16 hours, poured onto crushed ice, neutralized with ammonium hydroxide, and extracted with chloroform. The chloroform is evaporated to give 2-acetyl-8-acetamido-2,3,4,5-tetrahydro-1H-2-benzazepine.

22 g. of 2-acetyl-8-acetamido-2,3,4,5-tetrahydro-1H-2-benzazepine (0.1 mole) dissolved in acetic acid are added to a mixture of 48 g. of 90% fuming nitric acid (0.76 mole) and acetic acid (6 ml.) stirred in an ice bath so that the internal temperature does not exceed 15°C.

The mixture is stirred at 15°C for one hour, and poured into water to give 2-acetyl-8-acetamido-9-nitro-2,3,4,5-tetrhaydro-1H-2-benzazepine which is purified by chromatography and crystallization.

2-Acetyl-8-acetamido-9-nitro-2,3,4,5-tetrahydro-1H-2-benzazepine is hydrolyzed following the procedure of Example 1 to give 8-amino-9-nitro-2,3,4,5-tetrahydro-1H-2-benzazepine.

Example 9

2.1 g. of 8-amino-9-nitro-2,3,4,5-tetrahydro-1H-2-benzazepine (0.01 mole) in ethyl acetate (25 ml.) containing 2.1 g. of isopropenyl acetate (0.021 mole) is refluxed until acetylation is complete, and the mixture is evaporated to give 2-acetyl-8-amino-9-nitro-2,3,4,5-tetrahydro-1H-2-benzazepine.

Following the procedure of Example 4, 2-acetyl-8-amino-9-nitro-2,3,4,5-tetrahydro-1H-2-benzazepine is diazotized and converted into 2-acetyl-8-chloro-9-nitro-2,3,4,5-tetrahydro-1H-2-benzazepine which is hydrolyzed in 10% hydrochloric acid to give 8-chloro-9-nitro-2,3,4,5-tetrahydro-1H-2-benzazepine.

Example 10

2.68 g. of 2-acetyl-8-chloro-9-nitro-2,3,4,5-tetrahydro-1H-2-benzazepine (0.01 mole) are dissolved in boiling ethanol (40 ml.) and a solution of 6.3 g. of sodium hydrosulfite (0.03 mole) in water (40 ml.) is added dropwise. The mixture is refluxed, evaporated, and the residue is partitioned between dilute ammonium hydroxide and ether. Evaporation of the ether gives 2-acetyl-9-amino-8-chloro-2,3,4,5-tetrahydro-1H-2-benzazepine which is hydrolyzed to give 9-amino-8-chloro-2,3,4,5-tetrahydro-1H-2-benzazepine.

Example 11

Following the procedure of Example 4, and substituting an equivalent amount of sulfuric acid for hydrochloric acid, 2-acetyl-9-amino-8-chloro-2,3,4,5-tetrahydro-1H-2-benzazepine is diazotized and the resulting solution is warmed to 50°C until nitrogen evolution ceases. The mixture is cooled and extracted with ether to give 2-acetyl-8-chloro-9-hydroxy-2,3,4,5-tetrahydro-1H-2-benzazepine which is hydrolyzed to give 8-chloro-9-hydroxy-2,3,4,5-tetrahydro-1H-2-benzazepine.

Example 12

2.4 g. of 2-acetyl-8-chloro-9-hydroxy-2,3,4,5-tetrahydro-1H-2-benzazepine (0.01 mole) stirred in dimethylformamide (20 ml.) are treated with 0.24 g. of sodium hydride (0.01 mole), and then with 1.4 g of methyl iodide (0.01 mole), stirred, poured into water, and extracted with ether. The ether is washed and evaporated to yield 2-acetyl-8-chloro-9-methoxy-2,3,4,5-tetrahydro-1H-2-benzazepine which is hydrolyzed to give 8-chloro-9-methoxy-2,3,4,5-tetrahydro-1H-2-benzazepine.

Example 13

Following the procedure of Examples 4,6, and 7, 2-acetyl-8-amino-9-nitro-2,3,4,5-tetrahydro-1H-2-benzazepine is converted into the following compounds:

2-acetyl-8-fluoro-9-nitro-2,3,4,5-tetrahydro-1H-2-benzazepine
2-acetyl-8-bromo-9-nitro-2,3,4,5-tetrahydro-1H-2-benzazepine
2-acetyl-8-iodo-9-nitro-2,3,4,5-tetrahydro-1H-2-benzazepine
which upon hydrolysis yield respectively:
8-fluoro-9-nitro-2,3,4,5-tetrahydro-1H-2-benzazepine
8-bromo-9-nitro-2,3,4,5-tetrahydro-1H-2-benzazepine
8-iodo-9-nitro-2,3,4,5-tetrahydro-1H-2-benzazepine

Example 14

Following the procedure of Example 10, the following compounds:
2-acetyl-8-fluoro-9-nitro-2,3,4,5-tetrahydro-1H-2-benzazepine
2-acetyl-8-bromo-9-nitro-2,3,4,5-tetrahydro-1H-2-benzazepine
2-acetyl-8-iodo-9-nitro-2,3,4,5-tetrahydro-1H-2-benzazepine
respectively are converted into:
2-acetyl-9-amino-8-fluoro-2,3,4,5-tetrahydro-1H-2-benzazepine
2-acetyl-9-amino-8-bromo-2,3,4,5-tetrahydro-1H-2-benzazepine
2-acetyl-9-amino-8-iodo-2,3,4,5-tetrahydro-1H-2-benzazepine
and upon hydrolysis yield respectively:
9-amino-8-fluoro-2,3,4,5-tetrahydro-1H-2-benzazepine
9-amino-8-bromo-2,3,4,5-tetrahydro-1H-2-benzazepine
9-amino-8-iodo-2,3,4,5-tetrahydro-1H-2-benzazepine

Example 15

18.1 g. of 2-methyl-6-nitrobenzoic acid (0.1 mole) in acetone (200 ml.) are treated with 26 g. of N,N-diisopropylethylamine (0.2 mole) and 12.6 g. of dimethylsulfate (0.11 mole). The mixture is refluxed, evaporated, partitioned between chloroform and dilute hydrochloric acid, and the chloroform phase is evaporated to give methyl 2-methyl-6-nitrobenzoate.

19.5 g. of methyl 2-methyl-6-nitrobenzoate (0.1 mole) is dissolved in carbon tetrachloride (1.51.), 18.7 g. of N-bromosuccinimide (0.105 mole) are added, the mixture is refluxed, irradiated with a GE Sunlamp, and small amounts of dibenzoyl peroxide are added at frequent intervals until the reaction is complete. The mixture is filtered and evaporated to yield methyl 2-bromomethyl-6-nitrobenzoate.

2.7 g. of methyl 2-bromomethyl-6-nitrobenzoate (0.01 mole) in dimethylformamide (15 ml.) are added to a stirred mixture of 0.6 g. of sodium hydride (0.025 mole) and 2.2 g. of di-t-butyl malonate (0.01 mole) in dimethylformamide (15 ml.). The mixture is stirred for 30 minutes, poured onto crushed ice, extracted with ether, and the ether is evaporated to give di-t-butyl 2-[(2-carbomethoxy-3-nitro)benzyl]malonate.

The malonate is suspended in trifluoroacetic acid (30 ml.), warmed on a steam bath for 15 minutes, and evaporated to give 2-[(carbomethoxy-3-nitro)benzyl]malonic acid which is heated to effect decarboxylation and refluxed in 10% hydrochloric acid to give 3-[(2-carboxy-3-nitro)phenyl]-propionic acid.

Following the procedure of Example 4, the propionic acid is converted into 2-benzyl-9-nitro-2,3,4,5-tetrahydro-1H-2-benzazepine and the benzazepine is converted into 9-amino-2,3,4,5-tetrahydro-1H-2-benzazepine.

Example 16

2-Benzyl-9-nitro-2,3,4,5-tetrahydro-1H-2-benzazepine is converted into 9-nitro-2,3,4,5-tetrahydro-1H-2-benzazepine according to the procedure of Example 5.

Example 17

Following the procedure of Example 8, 9-amino-2,3,4,5-tetrahydro-1H-2-benzazepine is acetylated to give 2-acetyl-9-acetamido-2,3,4,5-tetrahydro-1H-2-benzazepine.

Following the procedure of Examples 8, 9 and 10, 2-acetyl-9-acetamido-2,3,4,5-tetrahydro-1H-2-benzazepine is converted into the following compounds:
2-acetyl-9-amino-8-nitro-2,3,4,5-tetrahydro-1H-2-benzazepine
2-acetyl-9-chloro-8-nitro-2,3,4,5-tetrahydro-1H-2-benzazepine
2-acetyl-8-amino-9-chloro-2,3,4,5-tetrahydro-1H-2-benzazepine
and upon hydrolysis they yield respectively:
9-amino-8-nitro-2,3,4,5-tetrahydro-1H-2-benzazepine
9-chloro-8-nitro-2,3,4,5-tetrahydro-1H-2-benzazepine
8-amino-9-chloro-2,3,4,5-tetrahydro-1H-2-benzazepine

Example 18

2.11 g. of 9-chloro-8-methoxy-2,3,4,5-tetrahydro-1H-2-benzazepine (as prepared in Example 2) (0.01 mole) is refluxed in 48% hydrobromic acid, cooled, and evaporated to give 9-chloro-8-hydroxy-2,3,4,5-tetrahydro-1H-2-benzazepine hydrobromide.

Example 19

Following the procedure of Example 13, 2-acetyl-9-amino-8-nitro-2,3,4,5-tetrahydro-1H-2, benzazepine is converted into 2-acetyl-9-fluoro-8-nitro-2,3,4,5-tetrahydro-1H-2,benzazepine
2-acetyl-9-bromo-8-nitro-2,3,4,5-tetrahydro-1H-2-benzazepine
2-acetyl-9-iodo-8-nitro-2,3,4,5-tetrahydro-1H-2-benzazepine and the acetyl compounds are hydrolyzed to yield respectively:
9-fluoro-8-nitro-2,3,4,5-tetrahydro-1H-2-benzazepine

9-bromo-8-nitro-2,3,4,5-tetrahydro-1H-2-benzazepine
9-iodo-8-nitro-2,3,4,5-tetrahydro-1H-2-benzazepine

Example 20

Following the procedure of Example 14, the following compounds:
2-acetyl-9-fluoro-8-nitro-2,3,4,5-tetrahydro-1H-2-benzazepine
2-acetyl-9-bromo-8-nitro-2,3,4,5-tetrahydro-1H-2-benzazepine
2-acetyl-9-iodo-8-nitro-2,3,4,5-tetrahydro-1H-2-benzazepine are converted into:
2-acetyl-8-amino-9-fluoro-2,3,4,5-tetrahydro-1H-2-benzazepine
2-acetyl-8-amino-9-bromo-2,3,4,5-tetrahydro-1H-2-benzazepine
2-acetyl-8-amino-9-iodo-2,3,4,5-tetrahydro-1H-2-benzazepine
which are hydrolyzed in 10% hydrochloric acid to yield respectively:
8-amino-9-fluoro-2,3,4,5-tetrahydro-1H-2-benzazepine
8-amino-9-bromo-2,3,4,5-tetrahydro-1H-2-benzazepine
8-amino-9-iodo-2,3,4,5-tetrahydro-1H-2-benzazepine

Example 21

10 g. of 2-acetyl-2,3,4,5-tetrahydro-1H-2-benzazepine (0.05 mole) are added to chlorosulfonic acid (100 ml.) at —40°C, stirred at 25°C for 48 hours, poured onto crushed ice, extracted with ethyl acetate, and evaporated to give 2-acetyl-8-chlorosulfonyl-2,3,4,5-tetrahydro-1H-2-benzazepine.

2.0 g. of the chlorosulfonyl compound (0.007 mole) were dissolved in tetrahydrofuran (10 ml.) and added to tetrahydrofuran (20 ml.) saturated with ammonia. The mixture was stirred for one hour, filtered, and the filter cake was crystallized from water to give 2-acetyl-8-sulfamyl-2,3,4,5-tetrahydro-1H-2-benzazepine: mp 223—224°C.

1.5 of the sulfamyl compound (0.006 mole) were refluxed in 10% hydrochloric acid for 8 hours, evaporated, and the residue was crystallized from isopropanol-ethyl acetate to give 8-sulfamyl-2,3,4,5-tetrahydro-1H-2-benzazepine hydrochloride: mp: 244—245°C.

Example 22

Following the procedure of Example 1, 7-chloroisatin is converted into 6-chloro-2,3,4,5-tetrahydro-1H-2-benzazepine which is acetylated according to the procedure of Example 8, and chlorosulfonated and then treated with ammonia following the procedure of Example 21 to give 2-acetyl-6-chloro-9-sulfamyl-2,3,4,5-tetrahydro-1H-2-benzazepine.

3 g. of the 6-chloro-9-sulfamyl compound (0.01 mole) in methanol (100 ml.) containing 250 mg. of 10% palladium-on-carbon and 1.3 g. of N,N-diisopropylethylamine (0.01 mole) are reduced with hydrogen until the theoretical amount is absorbed. The mixture is filtered, evaporated, and the residue is crystallized to give 2-acetyl-9-sulfamyl-2,3,4,5-tetrahydro-1H-2-benzazepine which is hydrolyzed according to the procedure of Example 21 to give 9-sulfamyl-2,3,4,5-tetrahydro-1H-2-benzazepine.

Example 23

0.9 g. of 2-acetyl-8-amino-9-chloro-2,3,4,5-tetrahydro-1H-2-benzazepine (0.0038 moles) in concentrated hydrochloric acid (3 ml.) is stirred, cooled to 0°C, and diazotized by addition of 0.3 g. of sodium nitrite (0.0044 moles) dissolved in water (2 ml.). The solution of the diazonium salt is added to a mixture of dioxane saturated with sulfur dioxide (3 ml.), 0.4 g. of potassium chloride, and 0.3 g. of cupric chloride dihydrate. The mixture is stirred at 25°C for one hour and then at 40—60°C for 30 minutes. The mixture is diluted with water, extracted with ethyl acetate, and the solvent is evaporated to give crude 2-acetyl-9-chloro-8-chlorosulfonyl-2,3,4,5-tetrahydro-1H-2-benzazepine. The chlorosulfonyl compound is treated with ammonia and hydrolyzed following the procedure of Example 21 to give 9-chloro-8-sulfamyl-2,3,4,5-tetrahydro-1H-2-benzazepine.

Example 24

Following the procedure of Example 23,the following compounds:
2-acetyl-8-amino-9-fluoro-2,3,4,5-tetrahydro-1H-2-benzazepine
2-acetyl-8-amino-9-bromo-2,3,4,5-tetrahydro-1H-2-benzazepine
2-acetyl-8-amino-9-iodo-2,3,4,5-tetrahydro-1H-2-benzazepine
are converted into the following compounds:
2-acetyl-9-fluoro-8-sulfamyl-2,3,4,5-tetrahydro-1H-2-benzazepine
2-acetyl-9-bromo-8-sulfamyl-2,3,4,5-tetrahydro-1H-2-benzazepine
2-acetyl-9-iodo-8-sulfamyl-2,3,4,5-tetrahydro-1H-2-benzazepine
which are hydrolyzed to yield respectively:
9-fluoro-8-sulfamyl-2,3,4,5-tetrahydro-1H-2-benzazepine
9-bromo-8-sulfamyl-2,3,4,5-tetrahydro-1H-2-benzazepine
9-iodo-8-sulfamyl-2,3,4,5-tetrahydro-1H-2-benzazepine

Example 25

Following the procedure of Example 23, 2-acetyl-8-amino-9-nitro-2,3,4,5-tetrahydro-1H-2-benzazepine is converted into 2-acetyl-8-chlorosulfonyl-9-nitro-2,3,4,5-tetrahydro-1H-2-benzazepine which is treated with ammonia to yield 2-acetyl-9-nitro-8-sulfamyl-2,3,4,5-tetrahydro-1H-2-benzazepine which upon hydrolysis gives 9-nitro-sulfamyl-2,3,4,5-tetrahydro-1H-2-benzazepine.

Example 26

2.7g. of 9-nitro-8-sulfamyl-2,3,4,5-tetrahydro-1H-2-benzazepine (0.01 mole) is reduced following the procedure of Example 4 to give 9-amino-8-sulfamyl-2,3,4,5-tetrahydro-1H-2-benzazepine.

Example 27

3.3 g. of 2-acetyl-8-chlorosulfonyl-9-nitro-2,3,4,5-tetrahydro-1H-2-benzazepine (0.01 mole) in tetrahydrofuran are treated with 4 g. of dibenzylamine (0.02 mole) to give 2-acetyl-8-N,N-dibenzylsulfamyl-9-nitro-2,3,4,5-tetrahydro-1H-2-benzazepine which is reduced with sodium hydrosulfite following the procedure of Example 10 to give 2-acetyl-9-amino-8-N,N-dibenzylsulfamyl-2,3,4,5-tetrahydro-1H-2-benzazepine which is diazotized and solvolyzed following the procedure of Example 11 to give 2-acetyl-8-N,N-dibenzylsulfamyl-9-hydroxy-2,3,4,5-tetrahydro-1H-2-benzazepine.

4.6 g. of the 8-N,N-dibenzylsulfamyl compound (0.01 mole) are refluxed in trifluoroacetic acid until dibenzylation is complete, and then hydrolyzed following the procedure of Example 4 to give 9-hydroxy-8-sulfamyl-2,3,4,5-tetrahydro-1H-2-benzazepine.

Example 28

4.6 g. of 2-acetyl-8-N,N-dibenzylsulfamyl-9-hydroxy-2,3,4,5-tetrahydro-1H-2-benzazepine (0.01 mole) are methylated following the procedure of Example 12 to give the 9-methoxy compound which is hydrolyzed following the procedure of Example 27 to give 9-methoxy-8-sulfamyl-2,3,4,5-tetrahydro-1H-2-benzazepine.

Example 29

Following the procedure of Example 23, the following compounds:

2-acetyl-9-amino-8-chloro-2,3,4,5-tetrahydro-1H-2-benzazepine
2-acetyl-9-amino-8-bromo-2,3,4,5-tetrahydro-1H-2-benzazepine
2-acetyl-9-amino-8-iodo-2,3,4,5-tetrahydro-1H-2-benzazepine
2-acetyl-9-amino-8-fluoro-2,3,4,5-tetrahydro-1H-2-benzazepine
2-acetyl-9-amino-8-nitro-2,3,4,5-tetrahydro-1H-2-benzazepine

are converted into, respectively:

8-chloro-9-sulfamyl-2,3,4,5-tetrahydro-1H-2-benzazepine
8-bromo-9-sulfamyl-2,3,4,5-tetrahydro-1H-2-benzazepine
8-iodo-9-sulfamyl-2,3,4,5-tetrahydro-1H-2-benzazepine
8-fluoro-9-sulfamyl-2,3,4,5-tetrahydro-1H-2-benzazepine
8-nitro-9-sulfamyl-2,3,4,5-tetrahydro-1H-2-benzazepine

Example 30

2.3 g. of 2-acetyl-8-chlorosulfonyl-2,3,4,5-tetrahydro-1H-2-benzazepine (0.008 mole) are dissolved in 75 ml. of warm glacial acetic acid and treated at 75°C with 11.25 g. of stannous chloride dihydrate (0.05 mole) in concentrated hydrochloric acid (10 ml.). The reaction mixture is allowed to cool to 25°C, stirred for 1.5 hours, and poured into 250 ml. of water containing concentrated hydrochloric acid. Crude 2-acetyl-8-mercapto-2,3,4,5-tetrahydro-1H-2-benzazepine is isolated by filtration and dried.

1.06 g. of the 2-acetyl-8-mercapto-2,3,4,5-tetrahydro-1H-2,benzazepine (0.0048 mole) are suspended in methanol (20 ml.), stirred in a nitrogen atmosphere, and 0.3 g. of sodium methoxide (0.0055 mole) is added followed by iodomethane (5 ml.). The mixture is stirred at 25°C for thirty minutes, evaporated, and partitioned between chloroform and water. The chloroform layer is washed, dried over sodium sulfate, and evaporated. The residue is chromatographed on silica gel eluted with ethyl acetate to give pure 2-acetyl-8-methylthio-2,3,4,5-tetrahydro-1H-2-benzazepine which is hydrolyzed following the procedure of Example 4 to give 8-methylthio-2,3,4,5-tetrahydro-1H-2-benzazepine.

Example 31

2.1 g. of 2-acetyl-8-methylthio-2,3,4,5-tetrahydro-1H-2-benzazepine (0.009 mole) dissolved in chloroform (20 ml.) are cooled to −20°C and treated with 1.6 of m-chloroperbenzoic acid (0.009 mole). The reaction mixture is allowed to warm to 25°C and it is washed with 10% aqueous sodium carbonate, dried over sodium sulfate, and evaporated to yield 2-acetyl-8-methylsulfinyl-2,3,4,5-tetrahydro-1H-2-benzazepine which is hydrolyzed as described in Example 4 to yield 8-methylsulfinyl-2,3,4,5-tetra-hydro-1H-2-benzazepine.

Example 32

2.1 g. of 2-acetyl-8-methylthio-2,3,4,5-tetrahydro-1H-2-benzazepine (0.009 mole) dissolved in chloroform (20 ml.) are treated with 3.1 g. of m-chloroperbenzoic acid (0.018 mole). The reaction mixture is stirred at 25°C, washed with 5% aqueous sodium carbonate, dried over sodium sulfate, and evaporated. The residue is chromatographed on silica gel eluted with ethanol-ethyl acetate to yield 2-acetyl-8-methylsulfonyl-2,3,4,5-tetrahydro-1H-2-benzazepine which is hydrolyzed as described in Example 4 to give 8-methylsulfonyl-2,3,4,5-tetrahydro-1H-2-benzazepine.

Example 33

11.8 g. of 2-acetyl-8-methylthio-2,3,4,5-tetrahydro-1H-2-benzazepine (0.05 mole) are dissolved in ethanol-free chloroform (250 ml.) containing 10 g. of cumene and stirred in the dark. Chlorine is passed through the solution for two days. The solvent is evaporated, and the residue is chromatographed on silica gel eluted with ethyl acetate to give 2-acetyl-8-trichloromethylthio-2,3,4,5-tetrahydro-1H-2-benzazepine.

5.8 g. of the 2-acetyl-8-trichloromethylthio-2,3,4,5-tetrahydro-1H-2-benzazepine (0.017 mole) are oxidized as described in Example 31, and the residue is chromatographed on silica gel eluted with ethyl acetate to yield 2-acetyl-8-trichloromethylsulfinyl-2,3,4,5-tetrahydro-1H-2-benzazepine which is hydrolyzed as described in Example 4 to give 8-trichloromethylsulfinyl-2,3,4,5-tetrahydro-1H-2-benzazepine.

Example 34

5.8 g. of 2-acetyl-8-trichloromethylthio-2,3,4,5-tetrahydro-1H-2-benzazepine (0.017 mole) are oxidized as described in Example 32 to yield 2-acetyl-8-trichloromethylsulfonyl-2,3,4,5-tetrahydro-1H-2-benzazepine which is hydrolyzed as described in Example 4 to yield 8-trichloromethylsulfonyl-2,3,4,5-tetrahydro-1H-2-benzazepine.

Example 35

5.4 g. of 2-acetyl-8-trichloromethylthio-2,3,4,5-tetrahydro-1H-2-benzazepine (0.016 mole) are added to a mixture of 15 g. of antimony trifluoride and 1.5 g. of antimony pentachloride, and heated and stirred at 90°C for one day. The resulting tar is partitioned between chloroform and 3N hydrochloric acid. The chloroform extract is dried over sodium sulfate and evaporated to give 2-acetyl-8-trifluoromethylthio-2,3,4,5-tetrahydro-1H-2-benzazepine which is hydrolyzed as described in Example 4 to give 8-trifluoromethylthio-2,3,4,5-tetrahydro-1H-2-benzazepine.

Example 36

5.2 g. of 2-acetyl-8-trifluoromethylthio-2,3,4,5-tetrahydro-1H-2-benzazepine (0.018 mole) are oxidized and chromatographed as described in Example 31 to yield 2-acetyl-8-trifluoromethylsulfinyl-2,3,4,5-tetrahydro-1H-2-benzazepine which is hydrolyzed as described in Example 4 to yield 8-trifluoromethylsulfinyl-2,3,4,5-tetrahydro-1H-2-benzazepine.

Example 37

5.2 g of 2-acetyl-8-trifluoromethylthio-2,3,4,5-tetrahydro-1H-2-benzazepine (0.018 mole) are oxidized as described in Example 32 and chromatographed on silica gel eluted with ethyl acetate to give 2-acetyl-8-trifluoromethyl sulfonyl-2,3,4,5-tetrahydro-1H-2-benzazepine which is hydrolyzed as described in Example 4 to give 8-trifluoromethylsulfonyl-2,3,4,5-tetrahydro-1H-2-benzazepine.

Example 38

3.7 g. of 8,9-dichloro-2,3,4,5-tetrahydro-1H-2-benzazepine (0.017 mole) in 5% aqueous sodium carbonate (400 ml.) are treated with 9.5 g. of potassium nitrosodisulfonate (0.034 mole) and stirred for 16 hours. The mixture is extracted with chloroform and the chloroform evaporated to give 8,9-dichloro-4,5-dihydro-3H-2-benzazepine which is purified by chromatography.

2.3 g. of 8,9-dichloro-4,5-dihydro-3H-2-benzazepine (0.011 mole) in ether (20 ml.) are stirred and treated with methylmagnesium iodide (0.029 mole) in ether (10 ml.). The mixture is treated with water, filtered, and extracted with ethyl acetate to give 8,9-dichloro-1-methyl-2,3,4,5-tetrahydro-1H-2-benzazepine which is purified by chromatography and crystallization of an appropriate salt.

Example 39

2.2 g. of 8,9-dichloro-2,3,4,5-tetrahydro-1H-2-benzazepine (0.01 mole) are refluxed in ethyl formate (20 ml.) and the mixture is evaporated to give 8,9-dichloro-2-formyl-2,3,4,5-tetrahydro-1H-2-benzazepine which is dissolved in tetrahydrofuran (20 ml.), added to 1M diborane in tetrahydrofuran (20 ml.) at 0°C, refluxed for one hour, cooled, treated with methanol, and evaporated. The residue is heated on the steam bath with 10% hydrochloric acid, the mixture is evaporated, and the residue is crystallized to give 8,9-dichloro-2-methyl-2,3,4,5-tetrahydro-1H-1-benzazepine hydrochloride.

Example 40

| Ingredients | Mg./Capsule |
|---|---|
| 8-sulfamyl-2,3,4,5-tetrahydro-1H-2-benzazepine | 150 mg. |
| Lactose | 150 mg. |

The above ingredients are mixed and filled into a hard gelatin capsule. One capsule is given three times a day.

Example 41

| Ingredients | Mg./Capsule |
|---|---|
| 8,9-Dichloro-2,3,4,5-tetrahydro-1H-2-benzazepine | 50 |
| Calcium sulfate dihydrate | 150 |
| Sucrose | 25 |
| Starch | 15 |
| Talc | 5 |
| Stearic Acid | 3 |

The sucrose, calcium sulfate and 2-benzazepine are thoroughly mixed and granulated with hot 10% gelatin solution. The wetted mass is passed through a No. 6 mesh screen directly onto drying trays. The granules are dried at 120°C and passed through a No. 20 mesh screen, mixed with starch, talc and stearic acid, and compressed into tablets.

Two tablets are administered three times a day.

**Claims**

1. A compound of the formula

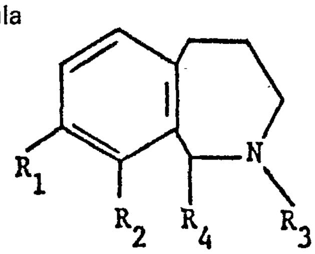

I

where $R_1$ and $R_2$, which are the same or different, are hydrogen, chloro, bromo, fluoro, iodo, trifluoromethyl, amino, nitro, hydroxy, lower alkyl, lower alkoxy, sulfamyl, methylthio, methylsulfinyl, methylsulfonyl, trichloromethylsulfinyl, trichloromethylsulfonyl, trifluoromethylthio, trifluoromethylsulfinyl, trifluoromethylsulfonyl, provided that both $R_1$ and $R_2$ are not both hydrogen; and $R_3$ and $R_4$, which are the same or different, are hydrogen or lower alkyl, each lower alkyl or lower alkoxy group containing from 1 to 3 carbon atoms; or a pharmaceutically acceptable acid addition salt thereof.

2. A compound according to Claim 1, characterized in that $R_3$ and $R_4$ are both hydrogen.

3. 8,9-Dichloro-2,3,4,5-tetrahydro-1H-2-benzazepine.

4. A compound according to Claim 2, characterized in that $R_1$ is sulfamyl and $R_2$ is hydrogen.

5. A compound according to Claim 2, characterized in that $R_1$ is sulfamyl and $R_2$ is chloro.

6. A pharmaceutical composition characterized in that it comprises a pharmaceutical carrier and a compound as claimed in any of Claims 1 to 5.

7. A process for preparing a compound according to Claim 1, characterized in that a compound of the formula:

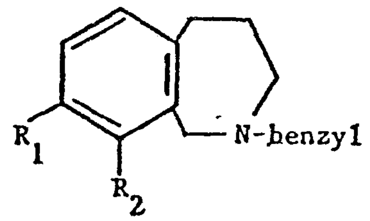

II

is debenzylated to yield an N-unsubstituted derivative where $R_3$ and $R_4$ are both hydrogen, and

(a) to prepare compounds where $R_3$ is lower alkyl, the product is N-alkylated by acylating the N-unsubstituted derivative followed by reduction of the resulting amide; or

(b) to prepare compounds where $R_4$ is lower alkyl, the N-unsubstituted derivative is oxidized to the corresponding imine followed by treatment with an organometallic agent;

and optionally forming a pharmaceutically acceptable acid addition salt thereof.

8. A process according to Claim 7, characterized in that $R_1$ and $R_2$ are both chloro.

**Patentansprüche**

1. Eine Verbindung der Formel I

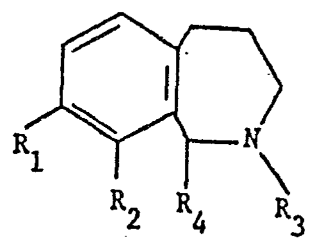

I

in der $R_1$ und $R_2$ gleich oder verschieden sind und ein Wasserstoff-, Chlor-, Brom-, Fluor- oder Jodatom, eine Trifluormethyl-, Amino-, Nitro- oder Hydroxylgruppe, einen Niederalkyl- oder Niederalkoxyrest, eine Sulfamyl-, Methylthio-, Methylsulfinyl-, Methylsulfonyl-, Trichlormethylsulfinyl-, Trichlormethylsulfonyl-, Trifluormethylthio, Trifluormethylsulfinyl- oder Trifluormethylsulfonylgruppe bedeuten, mit der Maßgabe, daß $R_1$ und $R_2$ nicht gleichzeitig ein Wasserstoffatom darstellen, und $R_3$ und $R_4$ gleich oder verschieden sind und ein Wasserstoffatom oder einen Niederalkylrest bedeuten, wobei jeder Niederalkyl- oder Niederalkoxyrest 1 bis 3 Kohlenstofatome enthält, oder deren pharmazeutisch verträgliches Säureadditionssalz.

2. Eine Verbindung nach Anspruch 1, dadurch gekennzeichnet, daß $R_3$ und $R_4$ je ein Wasserstoffatom bedeuten.

3. 8,9-Dichlor-2,3,4,5-tetrahydro-1H-2-benzazepin.

4. Eine Verbindung nach Anspruch 2, dadurch gekennzeichnet, daß $R_1$ eine Sulfamylgruppe und $R_2$ ein Wasserstoffatom bedeuten.

5. Eine Verbindung nach Anspruch 2, dadurch gekennzeichnet, daß $R_1$ eine Sulfamylgruppe und $R_2$ ein Chloratom bedeuten.

6. Arzneimittel, dadurch gekennzeichnet, daß es einen pharmazeutischen Trägerstoff und eine Verbindung gemäß den Ansprüchen 1 bis 5 enthält.

7. Verfahren zur Herstellung einer Verbindung nach Anspruch 1, dadurch gekennzeichnet, daß man von einer Verbindung der Formel II

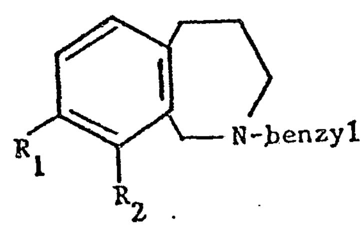

II

ausgehend die Benzylgruppe unter Bildung eines am Stickstoff nicht substituierten Derivats entfernt, wobei $R_3$ und $R_4$ beide ein Wasserstoffatom bedeuten, und

(a) zur Herstellung von Verbindungen, in denen $R_3$ einen Niederalkylrest bedeutet, das Produkt durch Acylierung des am Stickstoff nicht substituierten Derivats und anschließender Reduktion des erhaltenen Amids N-alkyliert; oder

(b) zur Herstellung der Verbindungen, in denen $R_4$ einen Niederalkylrest bedeutet, das am Stickstoff nicht substituierte Derivat zu dem entsprechenden Imin oxidiert und anschließend mit einer metallorganischen Verbindung behandelt;
und gegebenenfalls ein pharmazeutisch verträgliches Säureadditionssalz herstellt.

8. Verfahren nach Anspruch 7, dadurch gekennzeichnet, daß $R_1$ und $R_2$ je ein Chloratom bedeuten.

## Revendications

1. Composés de formule:

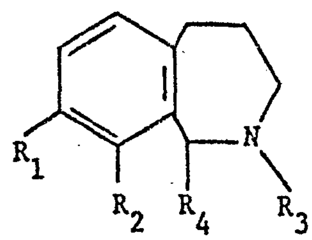

I

dans laquelle

$R_1$ et $R_2$, qui sont identiques ou différentes, représentent de l'hydrogène, des atomes de chlore, de brome, de fluor, d'iode, des groupes trifluorométhyle, amino, nitro, hydroxy, alkyle inférieur, alcoxy inférieur, sulfamyle, méthylthio, méthylsulfinyle, méthylsulfonyle, trichlorométhylsulfinyle, trichlorométhylsulfonyle, trifluorométhylthio, trifluorométhylsulfinyle, trifluorométhylsulfonyle, à condition que $R_1$ et $R_2$ ne représentent pas tous deux de l'hydrogène et

$R_3$ et $R_4$, qui sont identiques ou différents, représentent de l'hydrogène ou un groupe alkyle inférieur, chaque groupe alkyle inférieur ou alcoxy inférieur contenant de 1 à 3 atomes de carbone

ainsi que les sels d'addition d'acides pharmaceutiquement acceptables de ces composés.

2. Composés suivant la revendication 1, caractérisé en ce que $R_3$ et $R_4$ représentent tous deux de l'hydrogène.

3. 8,9-Dichloro-2,3,4,5-tétrahydro-1H-2-benzazépine.

4. Composés suivant la revendication 2, caractérisés en ce que $R_1$ représente un groupe sulfamyl et $R_2$ représente de l'hydrogène.

5. Composés suivant la revendication 2, caractérisée en ce que $R_1$ représente un groupe sulfamyle et $R_2$ représente un atome de chlore.

6. Composition pharmaceutique caractérisée en ce qu'elle comprend un véhicule pharmaceutique et un composé tel que revendiqué suivant l'une quelconque des revendications 1 à 5.

7. Procédé pour la préparation d'un composé suivant la revendication 1, caractérisé en ce qu'on débenzyle un composé de formule:

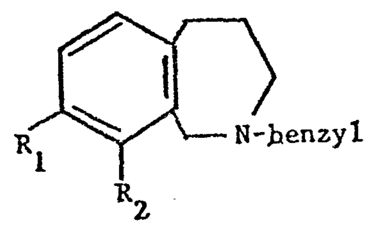

II

de façon à obtenir un dérivé N- non substitué dans lequel

$R_1$ et $R_2$ représentent tous deux l'hydrogène et

(a) afin de préparer des composés dans lesquels $R_3$ représente un groupe alkyle inférieur, on N-alkyle le produit par acylation du dérivé N- non substitué suivie de la réduction de l'amide résultant; ou

(b) afin de préparer des composés dans lesquels $R_4$ représente un groupe alkyle inférieur, on oxyde le dérivé N- non substitué en imine correspondante suivi d'un traitement par un agent organométallique;

et on forme éventuellement un sel d'addition d'acides pharmaceutiquement acceptable de ces composés.

8. Procédé suivant la revendication 7, caractérisé en ce que $R_1$ et $R_2$ représentent tous deux un atome de chlore.